(19) Europäisches Patentamt
European Patent Office
Office européen des brevets

(11) Veröffentlichungsnummer: **0 292 729 B1**

(12) **EUROPÄISCHE PATENTSCHRIFT**

(45) Veröffentlichungstag der Patentschrift: **22.04.92**

(51) Int. Cl.$^5$: **C08F 8/26**, C07K 1/04

(21) Anmeldenummer: **88106867.0**

(22) Anmeldetag: **06.05.88**

# (54) Neue Festphasen-Peptidsynthesemethoden.

(30) Priorität: **18.05.87 CH 1872/87**

(43) Veröffentlichungstag der Anmeldung:
**30.11.88 Patentblatt 88/48**

(45) Bekanntmachung des Hinweises auf die Patenterteilung:
**22.04.92 Patentblatt 92/17**

(84) Benannte Vertragsstaaten:
**AT BE CH DE ES FR GB GR IT LI LU NL SE**

(56) Entgegenhaltungen:
**DE-A- 2 319 495**
**US-A- 4 701 499**

(73) Patentinhaber: **BACHEM AG**
**Hauptstrasse 144**
**CH-4416 Bubendorf(CH)**

(72) Erfinder: **Mergler, Monika**
**Fraumattstr. 2**
**CH-4410 Liestal(CH)**
Erfinder: **Gosteli, Jacques**
**Anwilerstr. 10**
**CH-4059 Basel(CH)**
Erfinder: **Grogg, Peter**
**Moosmattstr. 5**
**CH-4416 Bubendorf(CH)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft einen neuartigen polymeren Träger, ein Verfahren zu dessen Herstellung und dessen Verwendung, zur Herstellung von Peptiden und deren Zwischenprodukte. Eine weiterer Gegenstand der Erfindung umfasst Derivate dieses polymeren Trägers, abgeleitet aus $N_\alpha$ - und allenfalls seitenkettengeschützten Aminosäuren.

Bekannt bisher sind konventionelle Verfahren zur Synthese von Peptiden, wie z.B. die ursprüngliche Flüssigphasen- und die von R.B. Merrifield entwickelte (R.B. Merrifield, J. Am. Chem. Soc. 85, 2149 [1963]) Festphasenmethode (Solid phase peptide synthesis [SPPS]).

In der Natur der chemischen Struktur begründet ist die zwingende Notwendigkeit, beim Aufbau von Peptiden später entfernbare Schutzgruppen zu verwenden. Diese trifft für beide erwähnten bekannten Synthesemethoden zu. Bei Anwendung der Flüssigphasenmethode können jedoch die Zwischenprodukte nach jedem Teilschritt aus der Lösung isoliert und gereinigt werden, wogegen dieses Prozedere der Reinigung bei der Festphasenmethode, bei der das wachsende Peptid stets an den unlöslichen polymeren Träger gebunden bleibt, nicht ausführbar ist. Nach vollzogener Synthese wird hier das Produkt - gleichzeitig mit der Entfernung der Seitenkettenschutzgruppen - vom Träger abgelöst. Das auf diese Weise erhaltene Syntheseprodukt muss, insbesondere, wenn es sich um ein längerkettiges Peptidprodukt handelt, einer aufwendigen Reinigung jeweils danach unterzogen werden.

Bislang verwendet wurde als polymerer Träger beispielsweise chlormethyliertes, granuliertes Polystyrol (R.B. Merrifield, loc. cit.), das mit 1 - 2 % Divinylbenzol (DVB) quervernetzt worden ist, und an welches die carboxylterminale Aminosäure in N-geschützter Form über die Carboxylgruppe als Ester gebunden ist. Nach vollendeter Synthese wird das erhaltene Endprodukt, gebunden an den Träger, unter sehr stark sauren Bedingungen vom Trägerharz unter gleichzeitiger Entfernung der Seitenkettenschutzgruppen abgelöst. Die zur Ausführung dieses Schrittes notwendigen drastischen Reaktionsbedingungen führen häufig zu unerwünschten Nebenreaktionen und daher, damit verbunden, zu unerwünschtem Produkteverlust. Des weiteren ist auf diesem Wege die Herstellung geschützter Fragmente (building blocks) für weitere Synthesen so nicht ausführbar.

In der EPA 0200404 wird eine weitere Festphasensynthese beschrieben, die endständige Peptidamide liefert, jedoch mit dem Nachteil, dass keine säureempfindlichen Schutzgruppen als Seitenkettenschutz verwendet werden können, falls geschützte Peptide oder -fragmente hergestellt werden sollen.

Ueberraschenderweise erlauben jedoch der erfindungsgemäss erhaltene und im folgenden beschriebene polymere Träger und die damit verbundene Arbeitstechnik zum ersten Mal die vorteilhafte Abspaltung eines Peptides in geschützter Form mit freier endständiger Carboxylgruppe unter äusserst milden Reaktionsbedingungen. Wichtigstes Merkmal der mit den erfindungsgemäss herstellbaren polymeren Trägerharzen ausführbaren Synthesemethode ist die Möglichkeit, in beliebiger Weise zu irgendeinem Zeitpunkt der Synthesefolge, ein seitenketten- und / oder aminoständig- geschütztes Peptid vom Träger abzulösen und es nach der Methode der Wahl als sogenanntes Fragment ("building block") weiter verwenden zu können. Diese mit dem neuen polymeren Trägerharz verbundene Technologie erweitert die strategischen Möglichkeiten der Peptidsynthese in bedeutendem Masse. Die mit dem erfindungsgemäss herstellbaren neuen polymeren Trägerharz verbundene vorteilhafte Methode gestattet es, in beliebiger Weise das End- oder Zwischenprodukt der ausgeführten Synthese unter sehr milden, schwach sauren Bedingungen so abzulösen, dass die Seitenkettenschutzgruppen und gewünschtenfalls die N-terminalen Schutzgruppen nicht abgespalten werden und damit intakt bleiben, so dass das so erhaltene Syntheseprodukt direkt weiter verwendet werden und an der freigesetzten Carboxylendgruppe durch weiteren Aufbau in bekannter Weise verlängert werden kann.

Ueberraschenderweise wurde gefunden, dass das im nachfolgenden beschriebene Polymerharz der Formel I

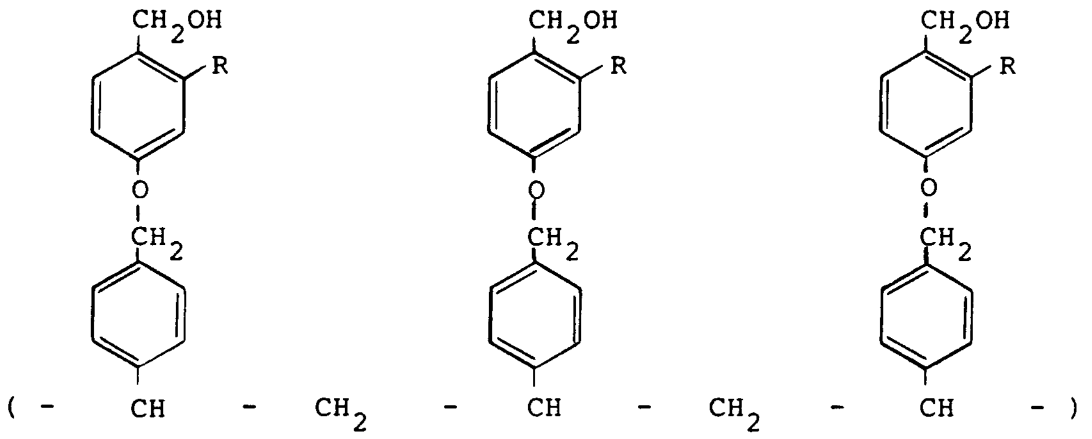

(I)

in welcher R eine Niederalkoxy-, Niederalkylthiogruppe oder eine

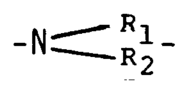

Gruppe bedeutet, in welcher $R_1$ und $R_2$ Niederalkyl bedeuten, welches mit 0,5 - 2 % eines gegebenenfalls durch Halogen oder auch Niederalkyl substituierten Divinylbenzols vernetzt ist, als Trägerharz für die neue Synthesemethode besonders geeignet ist.

Der Ausdruck "nieder" bedeutet, dass entsprechend definierte Gruppen oder Verbindungen vor- und nachstehend bis mit 7, vorzugsweise bis mit und mit 4 Kohlenstoffatome enthalten.

Der Substituent R als Niederalkoxy steht z.B. für Methoxy, Aethoxy, n-Propyloxy, Isopropyloxy, n-Butyloxy, Isobutyloxy oder tert-Butyloxy, ferner n-Pentyloxy, Isopentyloxy, Neopentyloxy, n-Hexyloxy, Isohexyloxy oder auch n-Heptyloxy.

Niederalkylthio ist z.B. Methylthio, Aethylthio, n-Propylthio, Isopropylthio, n-Butylthio, Isobutylthio oder tert-Butylthio, n-Pentylthio, n-Hexylthio oder auch n-Heptylthio.

Im Substituent R können $R_1$ und $R_2$ als Diniederalkylreste in der Diniederalkylaminogruppe beispielsweise Methyl, Aethyl, n-Propyl, Isopropyl, n-Butyl, Isobutyl, sek.-Butyl, tert-Butyl, n-Pentyl, Isopentyl, Neopentyl, n-Hexyl oder auch n-Heptyl bedeuten.

Der Substituent R als Diniederalkylamino ist z.B. N, N-Dimethylamino, N-Aethyl-N-methylamino oder N, N-Diäthylamino.

Halogen kann z.B. Chlor, Brom, Jod oder auch Fluor bedeuten.

Von besonderem Interesse sind Trägerharze aus einem Polymeren der Formel I, in der R Niederalkoxy bedeuten, welche mit 1 - 2 % des 1,4-unsubstituierten Divinylbenzols vernetzt ist.

Besonders hervorzuheben sind jedoch Trägerharze aus einem Polymeren der Formel I, in der R Methoxy bedeutet, welches mit 1 - 2 % des unsubstitituierten 1,4-Divinylbenzols vernetzt ist.

Polymere Verbindungen der Formel I werden erhalten, indem das von R.B. Merrifield bekannte Chlormethylpolystyrolharz der Formel II,

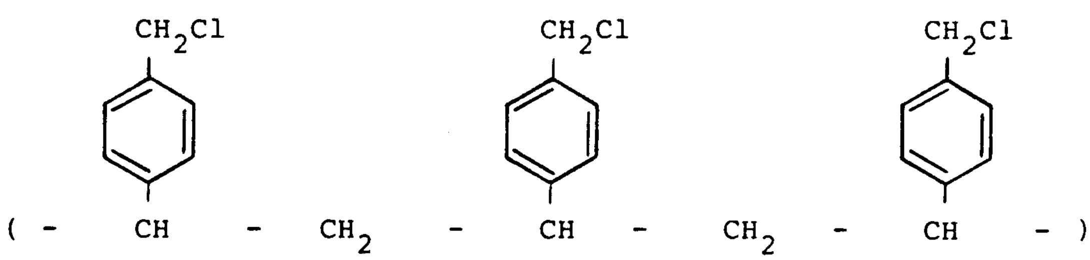

(II)

welches mit 0,5 - 2 % eines gegebenenfalls durch Halogen oder Niederalkyl substituierten Divinylbenzols vernetzt ist,unter basischen Bedingungen mit einem 4-Hydroxymethyl-3-R-subst.-phenol umsetzt, wobei die phenolische Hydroxygruppe veräthert wird.

Beispielsweise ist das als Ausgangsmaterial verwendete 4-Hydroxymethyl-3-methoxyphenol der Formel III,

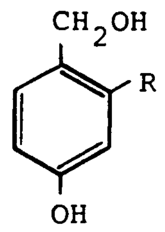

(III)

welches als Anker (linker) zwischen den Polymeren der Formel II und dem aufzubauenden Peptid bzw. Peptidbaustein fungiert neu und kann aus dem von R.C. Sheppard und B. Williams in Int. Journ. Pept. Prot. Res. 20, 451 (1982) beschriebenen 3-Methoxy-4-formylphenol durch Reduktion erhalten werden. Die anderen neuen 3-R-subst. Phenole der Formel III werden analog hergestellt.

Die Verätherung der phenolischen Hydroxygruppe kann in alkalischem Medium, beispielsweise in Gegenwart von Natronlauge, Natriumcarbonat, -hydrogencarbonat oder auch in Gegenwart eines Alkali- oder auch Erdalkalialkoholats, wie z.B. Natriummethylat oder auch -äthylat wie von S.S. Wang in J. Am, Chem. Soc. 95, 1328 (1973) beschrieben, ausgeführt werden.

An das so erhaltene Trägerharz, ein Copolymerisat eines Polymeren der Formel I mit einem gegebenenfalls substituierten Divinylbenzol, wird die carboxylendständige Aminosäure des zu synthetisierenden Peptids, welche an der Aminofunktion durch eine Aminoschutzgruppe gechützt ist, insbesondere mit einer basisch abspaltbaren Schutzgruppe geschützt ist, über die Carboxylgruppe durch Veresterung kondensiert. Dies Veresterung kann nach an sich bekannten Methoden durch Aktivierung der Säurefunktion erfolgen.

Die Carboxylgruppe kann beispielsweise durch Ueberführung in ein Säureazid, -anhydrid, -imidazolid, -isoxazolid oder einen aktivierten Ester, oder durch Reaktion mit einem Carbodiimid, wie z.B. N, N′-Dicyclohexylcarbodiimid, gegebenenfalls unter Zusatz von 4-tert. Aminopyridin, N-Hydroxysuccinimid, einem unsubstituierten 1-Hydroxybenzotriazol oder z.B. durch Halogen, Methyl oder Methoxy substiuierten 1-Hydroxybenzotriazol oder 4-Hydroxybenzo-1,2,3-triazin-3-oxid, oder insbesondere von N-Hydroxy-5-norbornen-2,3-dicarboximid, oder mit N, N′-Carbonyldiimidazol aktiviert werden. Als die gebräuchlichste Kupplungsmethode ist die Carbodiimidmethode unter Verwendung von 4-tert. Aminopyridin zu nennen.

Zur Bildung von aktivierten Estern, wie oben erwähnt, eignen sich z.B. gegebenfalls durch elektronenanziehende Substituenten substituierte Phenole und Thiophenole, wie z.B. Phenol, Thiophenol, Thiokresol, p-Nitro-thiophenol, 2,4,5- und 2,4,6-Trichlorphenol, Pentachlorphenol, o- und p- Cyanphenol, weiter z.B. N-Hydroxysuccinimid, N-Hydroxy-phthalimid oder ähnlich aus der Peptidchemie bekannte Aktivierungskomponenten, vgl. Houben-Weyl: Methoden der Org. Chemie: 4. Auflage, Bd. 15/I und II, E. Wünsch: Synthese von Peptiden (Georg Thieme Verlag, Stuttgart, 1974).

Beispielsweise wird die mit einer Aminoschutzgruppe versehene Aminosäure oder der Peptidbaustein an das Trägerharz in Anwesenheit von Dicylohexylcarbodiimid (DCC) und 4-tert. Aminopyridin in einer Zeitspanne von 2 - 24 Stunden, vorzugsweise jedoch innerhalb von 12 Stunden bei einer Temperatur von 0° - 50° C, vorzugsweise bei einer Temperatur von 20° - 25° , in einem Lösungsmittel wie z.B. Methylenchlorid oder Dimethylformamid oder einem Gemisch aus beiden gekuppelt.

Trägt diese anzukondensierende Aminosäure, wie auch die gegebenenfalls noch anzukondensierenden Aminosäuren eine Funktion in der Seitenkette, wie z.B. eine Carboxy-, Amino-, Hydroxy-, Amino-, Mercapto- oder auch Guanidinofunktion, so wird diese bevorzugt durch eine Gruppe geschützt, die bei der Ablösung der Aminosäure oder auch zu einem späteren Zeitpunkt des Peptids vom Harz nicht abgespalten wird.
Die engere Auswahl der Schutzgruppen richtet sich nach dem spezifischen Zweck, wobei man insbesondere bei mehreren zu schützenden funtionellen Gruppen zweckmässige Kombinationen wählen muss.

Unter einer Carboxyl-Schutzgruppe ist eine in der Peptidchemie üblicherweise zur Anwendung kommende Schutzgruppe zu verstehen. Solche Schutzgruppen sind leicht, d.h. ohne dass unerwünschte Nebenreaktionen stattfinden, beispielsweise solvolytisch, wie z.B. acidolytisch oder basisch, hydrogenolytisch, reduktiv, photolytisch oder auch unter physiologischen Bedingungen, abspaltbar, wobei in der Regel die Schutzgtruppen durch Wasserstoff ausgetauscht werden. Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in "Methoden der Organischen Chemie", Houben-Weyl, 4. Auflage, Bd. 15/I, Georg Thieme

Verlag, Stuttgart, (1974).

So werden Carboxygruppen in dieser Synthese beispielsweise durch Veresterung geschützt. Zur Veresterung besonders geeignet sind z.B. niedere substituierte Alkanole wie 2,2,2-Trichloräthanol, tert. -Butanol oder Benzoylmethanol oder auch Benzylalkohol.

Besonders geeignete Schutzgruppen sind die tert.-Butylgruppe und die Benzylgruppe.

Eine Aminoschutzgruppe ist in erster Linie eine Acylgruppe, wie Acyl einer aliphatischen, aromatischen oder araliphatischen Carbonsäure, insbesondere Niederalkanoyl, z.B. Acetyl oder Propionyl, oder Aroyl, z.B. Benzoyl, oder Acyl der Ameisensäure oder eines Kohlensäurehalbderivates, z.B. -esters, wie Formyl, Niederalkoxycarbonyl, z.B. Benzyloxycarbonyl.

Die Abspaltung eines als Aminoschutzgruppe verwendeten Acylrestes kann in an sich bekannter Weise, z.B. durch Solvolyse, in erster Linie mittels Alkoholyse, ferner mittels Hydrolyse acidolytisch oder basisch erfolgen. Die alkoholytische Abspaltung eines Acylrestes kann z.B. in Gegenwart eines stark basischen Mittels, bei erhöhter Temperatur, z.B. bei etwa 50° C bis etwa 120° C, erfolgen. Dabei verwendet man insbesondere einen Niederalkanol, z.B. n-Butanol oder Aethanol, und als starke Base ein Alkalimetall-, z.B. Natrium- oder Kalium-niederalkanolat, z.B. -n-butylat oder -äthylat, oder ein Alkalimetall-hydroxid, z.B. Natrium- oder Kaliumhydroxid.

Aminoschutzgruppen, beispielsweise Niederalkoxycarbonylgruppen, wie tert.-Butyloxycarbonyl, lassen sich besonders schonend acidolytisch, z.B. durch Behandeln mit Trifluoressigsäure, abspalten. Eine weitere, besonders schonend abspaltbare Aminoschutzgruppe ist eine Aethoxycarbonylgruppe, die in $\beta$-Stellung eine mit drei Kohlenwasserstoffresten substituierte Silylgruppe, wie Triphenylsilyl-, Dimethyl-butylsilyl- oder vor allem Trimethylsilygruppe, trägt. Eine solche $\beta$-(Trimethylsilyl)-äthoxycarbonylgruppe bildet mit der zu schützenden Aminogruppe eine entsprechende $\beta$-Trimethylsilyläthoxycarbonylaminogruppe, welche sich unter milden Bedingungen durch Einwirkung von Fluoridionen abspalten lässt. Als Fluoridionen-abgebende Reagentien kommen beispielsweise Fluoride quaternärer organischer Basen, wie Tetraäthylammoniumfluorid in Frage.

Eine besonders für den Peptidaufbau verwendet $\alpha$-Aminoschutzgruppe ist die 9-Fluorenylmethyloxycarbonylgruppe, welche basisch abgespalten werden kann, z.B. mittels Alkalimetallhydroxyden, -carbonaten oder auch -bicarbonaten, besonders schonend jedoch mit organischen Basen, wie z.B. Piperidin unter milden Bedingungen.

Als $\epsilon$-Aminoschutzgruppe kann jede in der Peptid-Chemie übliche Amino-Schutzgruppe wie oben angegeben, verwendet werden. Soll jedoch nach jedem Syntheseschritt die $\alpha$-Aminoschutzgruppe zur Fortsetzung der Synthese abgespalten werden, beispielsweise basisch oder auch hydrogenolytisch, verwendet man zum Schutz der $\epsilon$-Aminogruppe vorteilhafterweise zur Acylierung die tert.-Butoxycarboxylgruppe, die nach Abschluss der gesamten Synthese durch saure Hydrolyse abgespalten wird.

Als Hydroxyl-Schutzgruppe können alle zu diesem Zwecke in der Peptid-Chemie gebräuchlichen Gruppen verwendet werden, vgl. das oben zitierte Werk (Houben-Weyl): Bevorzugt sind acidolytisch abspaltbare Gruppen, wie 2-Tetrahydropyranyl und ganz besonders tert.-Butyl, sowie auch tert.-Butoxycarbonyl. Ferner kann man aber auch reduktiv abspaltbare Hydroxyl-Schutzgruppen verwenden, z.B. Benzyl- und Benzyloxycarbonylgruppen, die im aromatischen Teil durch Halogen, Nitro und / oder Niederalkoxy substituiert sein können, bzw. Niederalkanoylreste wie Acetyl, oder Aroylreste, wie Benzoyl. Insbesondere kommen als Schutzgruppe leicht solvolysierbare oder auch hydrogenolysierbare Aether- oder Acyloxygruppen wie oben angedeutet in Frage.

Eine leicht solvolysierbare oder auch hydrogenolysierbare Aether- oder Acyloxygruppe ist z.B. eine durch Solvolyse, einschliesslich Hydrolyse, Acidolyse oder Alkoholyse oder mittels Reduktion, einschliesslich Hydrogenolyse abspaltbare Aether- oder auch Acyloxygruppe.

Eine durch Solvolyse abspaltbare Acyloxygruppe ist beispielsweise eine Acyloxygruppe, in der der Acylteil der Rest einer organischen Carbonsäure, z.B. Niederalkanoly, wie Acetyl, Halogenniederalkanoyl, wie Halogenacetyl, z.B. Chloracetyl, oder Carbamoyl, oder Aroyl, wie Benzoyl, ferner der Acylrest den Rest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl oder Aroylmethoxycarbonyl, z.B. Phenacyloxycarbonyl, ferner eine gegebenenfalls substituierte 1-Polyphenyl-niederalkylgruppe, worin Substituenten des Phenylteils, z.B. Niederalkyl oder Niederalkoxy, z.B. Methyl oder Methoxy sein können, und in erster Linie Trityl oder auch einen Organosilylrest, insbesondere Trimethylsilyl bedeuten.

Eine solvolytisch abspaltbare Aethergruppe ist z.B. Niederalkoxy, beispielsweise Methoxy oder Aethoxy, oder eine 1-Phenylniederalkoxygruppe, wie z.B. Benzyloxy. Diese Reste können durch Niederalkoxy, z.B. Methoxy oder Aethoxy, oder auch Niederalkoxyäthoxy, z.B. Methoxyäthoxy substituiert sein.

Benzyloxyreste als abspaltbare Aethergruppen können gegebenenfalls durch eine oder auch mehrer Substituenten, wie z.B. Niederalkyl, beispielsweise Methyl, Aethyl, Isopropyl oder n-Propyl, Halogen, beispielsweise Chlor oder Brom, oder auch Niederalkoxy, wie z.B. Methoxy oder Aethoxy, substituiert sein. Diese Substituenten sitzen vorzugsweise in ortho- oder auch in para-Stellung.

Ebenfalls solvolytisch, insbesondere hydrolytisch oder alkoholytisch in saurem Medium abspaltbar sind ihrerseits in $\alpha$-Stellung durch eine Aethergruppe substituierte aliphatische Aethergruppen, wie Aethoxymethoxy, Butoxymethoxy oder 1-Aethoxyäthoxy, und insbesondere analoge cyclische Reste, z.B. 1-Oxacycloalkan-2-yloxygruppen, vor allem Tetrahydropyran-2-yloxy, ferner z.B. auch 4-Methoxytetrahydropyran-4-yloxy.

Wird die Solvolyse der Aether- oder auch Acyloxygruppen $R'$ und / oder $-OZ'$ hydrolytisch ausgeführt, so wird diese je nach Art der abspaltbaren Gruppen in Gegenwart einer organischen Säure, wie p-Toluolsulfonsäure oder einer Mineralsäure, wie Chlorwasserstoff- oder Schwefelsäure ausgeführt. Wird die Solvolyse mittels einer der obengenannten Säuren in einem Alkohol, beispielsweise mittels p-Toluolsulfonsäure in Aethylalkohol ausgeführt, so erfolgt die Solvolyse alkoholytisch.

Acidolytisch abspaltbar sind Acyloxygruppen, in denen der Acylteil ein Acylrest von Halbestern der Kohlensäure, wie z.B. tert.-Niederalkoxycarbonyl, oder gegebenenfalls substituiertes Diphenylemthoxycarbonyl bedeutet. Ferner können auch Aethergruppen wie z.B. tert.-Niederalkoxygruppen acidolytisch abgespalten werden. Die acidolytische Abspaltung kann durch Behandeln mit geeigneten starken organischen Carbonsäuren, wie gegebenenfalls durch Halogen, insbesondere Fluor, substituierten Niederalkansäuren, in erster Linie Trifluoressigsäure (gegebenenfalls in Gegenwart eines scavengers, wie Anisol), sowie mit Ameinsensäure erfolgen. Die obigen Reaktionen werden, wenn nicht schon vorher erwähnt, in Gegenwart eines Lösungsmittels oder Lösungsmittelgemiches durchgeführt, wobei geeignete Reaktionsteilnehmer auch als solche dienen können.

Als Mercapto-Schutzgruppe kann man alle zu diesem Zwecke in der Peptid-Chemie gebräuchlichen Gruppen verwenden, wobei die Mercapto-gruppen insbesondere durch geeignete Acylierung, Alkylierung oder Disulfidbildung geschützt werden.

Zur Acylierung geeignet ist z.B. der Acetyl- oder Benzoylrest, eine Niederalkyl- (z.B. Aethyl-) -carbamoyl, oder eine gegebenenfalls substituierte Benzyloxycarboxyl-Gruppe (Carbobenzoxygruppe). Zur Alkylierung geeignet sind z.B. der tert. Butyl-, Isobutyloxymethyl-, Benzylthiomethyl- oder Tetrahydropyranylrest oder gegebenenfalls durch Halogen, Niederalkoxy oder Nitro substituierte Anylmethylrest wie Benzyl, p-Methoxybenzyl, Diphenylmethyl, Dimethoxybenhydryl oder ganz besonders Trityl, sowie auch Phenylcyclohexyl, p-Methoxyphenylcyclohexyl oder auch Thienyl(2)cyclohexyl. Ferner können auch Acylaminomethylgruppen, insbesondere die Acetylaminomethylgruppe verwendet werden.

Geeignete Schutzgruppen für die Carbonamidfunktion sind methoxy-substituierte Benzylgruppen sowie die Diphenylmethylgruppe einschliesslich verschiedener Varianten gemäss "Methoden der Organischen Chemie", Houben-Weyl, 4. Auflage, Bd. 15/1 Georg Thieme Verlage (1974), S. 453 - 467. Insbesondere sind 4-Methoxy-benzyl, 2,4-Dimethoxy-benzyl- und 2,4,4-Trimethoxy-benzylgruppen als Schutzgruppen zur Markierung der Carbonamidfunktion geeignet. Als Diphenylmethylgruppen eignen sich insbesondere die 4,4'-Dimethyl- oder 4,4'-Dimethoxy-diphenylmethylgruppe zum Schutze der Carbonamidfunktion. Alle genannten Amid-Schutzgruppen können acidolytisch abgespalten werden, wobei zur Erleichterung der Abspaltung ein "Kationen-Fänger" wie z.B. Anisol zugesetzt werden kann.

Als Guanidinoschutzgruppe können die in der Literatur, beispielsweise in "Protective Groups in Organic Chemistry", Plenum Press, London, New York (1973), in "The Peptides", Vol. I, Schröder and Lübke (1965), sowie in "Chemie und Biochemie der Aminosäuren, Peptide und Proteine", S.99 (Lübke, Schröder und Kloss), Georg Thieme Verlag (1973) ferner auch in "Methoden der Organischen Chemie", Houben-Weyl, 4. Auflage, Bd. 15/1, George Thieme Verlag, Stuttgart, 1974 beschriebenen Schutzgruppen verwendet werden. Beispilsweise kann die von A. Konel u. E.L. Kennaway, H. Bergmann und L. Zervas erarbeitete Nitrierung der Guanidinogruppe und in "Methoden der Organischen Chemie", Houben-Weyl, 4. Auflage, Bd. 15/1, George Thieme Verlag (1974), S. 507 - 511 beschriebene Methode oder die Methode der Protonierung oder auch Acylierung gemäss Houben-Weyl (ebenda) verwendet werden. Beispielsweise kommen als Schutzgruppe für die Guanidinogruppe z.B. Nitro, Tosyl, p-Methoxybenzolsulfonyl, Carbobenzoxy, Isobornyloxycarbonyl, Adamantyloxycarbonyl und insbesondere für die Guanidinogruppe im Arginin die 4-Methoxy-2,3,6-Trimethylbenzolsulfonylgruppe, die Mesitylensulfonylgruppe und die tert.-Butoxycarbonylgruppe als besonders vorteilhafte Schutzgruppen in Frage. (Prinzip: bekannte säurelabile Schutzgruppen).

Das hergestellte Aminosäurederivat mit dem erfindungsgemäßen Harz, in der die Aminogruppe und gegebenenfalls andere funktionelle Gruppen geschützt sind, kann als Ausgangsmaterial zur Herstellung gewünschter Peptide oder solcher geschützter Peptide, bzw. Fragmente verwendet werden, deren carboxylendständige Aminosäure der harzgebundenen Aminosäure entspricht. Im gebildeten Produkt kann man

die terminale Aminogruppe freisetzen und dieses Produkt, bestehend aus den Komponenten das neuen Harzes und einer Aminosäure oder eines Peptides, wieder mit einer aktivierten Komponente, d.h. einer Aminosäure- oder einem Peptid mit aktivierter Carboxylgruppe und geschützter α-Aminogruppe, umsetzen. Die Synthese kann auf diese Weise beliebig fortgesetzt werden.
Die hierbei erhaltenen Fragmente, welche sich aus der Komponente des neuen Trägerharzes und einer Aminosäure oder einem Peptid zusammensetzen, sind ebenfalls neu und als Zwischenprodukte für weitere Synthesen verwendbar.

Die so erhaltenen Fragmente, bestehend aus der Komponente des neuen Trägerharzes und einer Aminosäure oder einem Peptid, der Formel IV,

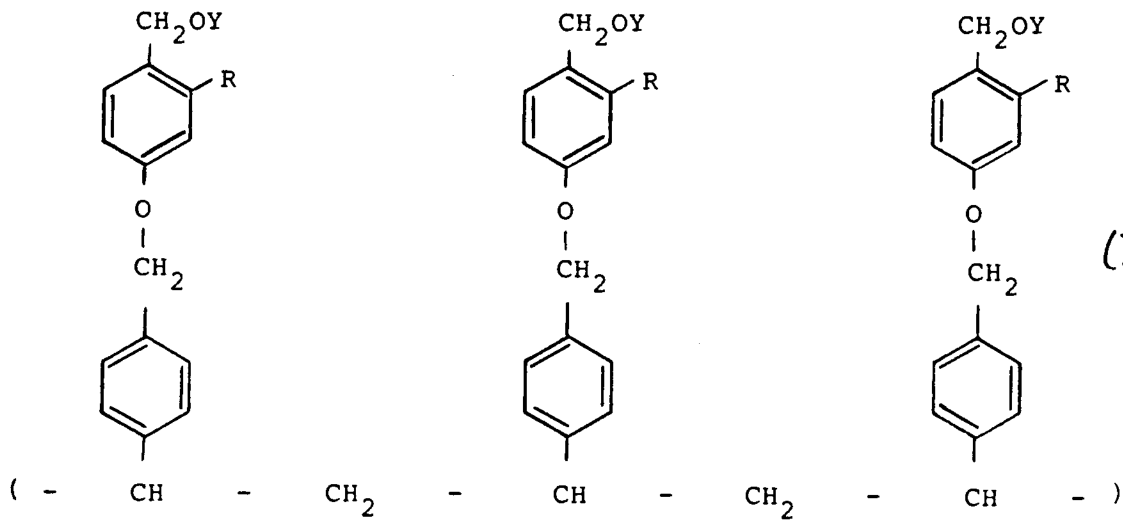

(IV)

in welcher R die unter der Formel I angegebene Bedeutung hat und Y den Rest einer Aminosäure oder den Rest eines Peptides, welche(s) mit der endständigen Carboxylgruppe an das Trägerharz kondensiert ist (Ester), in welcher die funktionellen Gruppen der Aminosäure oder der Aminosäuren als Peptidbausteine gegebenenfalls geschützt sind, sind auch Gegenstand der Erfindung.

Von besonderem Interesse sind Fragmente der Formel IV, in welcher R die unter der Formel I angegebene Bedeutung hat und Y den Rest einer als Peptidbaustein natürlich vorkommenden α-Aminosäureder L-Reihe und deren nahe verwandte Analoge, wie insbesondere der Enantiomeren der unnatürlichen D-Reihe oder der Rest eines Peptides bedeuten, in welchem die funktionellen Gruppen die Aminosäuren gegebenfalls durch Schutzgruppen substituiert sind.

Von ganz besonderem Interesse sind Fragmente der Formel IV, in der R Niederalkoxy, insbesondere Methoxy und Y den Rest einer natürlich vorkommenden α-Aminosäure bedeutet.

Besonders vorteilhaft können die geschützten Peptide unter sehr milden Bedingungen, insbesondere schwach sauren Bedingungen so abgelöst werden, dass die Seitenkettenschutzgruppen und gewünschtenfalls die N-terminale Schutzgruppe intakt bleiben, so dass das so gewonnene Produkt direkt weiter verwendet werden kann, indem an der freigewordenen Carboxylendgruppe durch weiteren Aufbau verlängert werden kann. Auf diese Weise können beispielsweise Peptide und auch Polypeptide synthetisiert werden, die 40, 50, 60 und auch mehr Aminosäuren enthalten können.

Als Bausteine können alle zugänglichen Aminosäuren, wie sie beispielsweise von K.D. Koppe in "Peptides and Amino Acids", (W.A. Benjamin, Inc., New York, 1966, p.4-7) beschrieben werden, insbesondere die als Peptid-Bausteine vorkommenden α-Aminosäuren der L-Reihe und deren nahe verwandte Analoge, wie insbesondere die Enantiomeren der "unnatürlichen" D-Reihe.

Wenn nicht anders angegeben, beziehen sich die Kurzbezeichnungen der Aminosäurereste auf Reste der α-Aminosäuren der L-Reihe, welche in der Natur vorkommen.

Die erhaltenen Fragmente, welche sich aus der Komponente des Trägerharzes und einer Aminosäure oder einem Peptid zusammensetzen, können besonders vorteilhaft für weitere Peptidsynthesen verwendet werden.

In den nachfolgenden Beispielen wird die Erfindung illustriert, jedoch durch diese nich eingeschränkt, Temperaturen werden in Celsiusgraden angegeben, als Abkürzungen, z.B. zur Bezeichnung von Aminosäuren, Peptiden, Schutzgruppen, Lösungsmittel, etc., werden die üblichen, z.B. die in "Synthesen von Peptiden" (Herausgeber: E. Wünsch), Bd. XV der "Methoden der Org. Chemie" (Houben-Weyl) (1974); G.

Thieme, Stuttgart, zusammengestellte Kurzbezeichnungen verwendet.

Insbesondere werden folgende Abkürzungen verwendet:

| | |
|---|---|
| Alanin | Ala |
| α-Amino-buttersäure | Abu |
| Arginin | Arg |
| Asparagin | Asn°) |
| Asparaginsäure | Asp |
| Cystein | Cys |
| α,γ-Diamino-buttersäure | Dab |
| α,β-Diamino-propionsäure | Dap |
| Glutamin | Gln° |
| Glutaminsäure | Glu |
| Glycin | Gly |
| Histidin | His |
| δ-Hydroxy-lysin | Hyl |
| γ-Hydroxy-prolin | Hyp |
| β-Hydroxy-prolin | 3Hyp |
| Isoleucin | Ile |
| Leucin | Leu |
| Lysin | Lys |
| Methionin | Met |
| Ornithin | Orn |
| Phenylalanin | Phe |
| Prolin | Pro |
| Serin | Ser |
| Threonin | Thr |
| Tryptophan | Trp |
| Tyrosin | Tyr |
| Valin | Val |

**Weitere α-Aminosäuren**

| | |
|---|---|
| Alliin | All |
| C-Allyl-glycin | Alg |
| α-Amino-adipinsäure | Aad |
| 1-Amino-cyclohexancarbonsäure | Ach |
| 1-Amino-cyclopentancarbonsäure | Acp |
| 1-Amino-cyclopropancarbonsäure | Apr |
| α-Amino-isobuttersäure | Aib |
| Amino-malonsäure | Ama |
| 2-Amino-3-Methyl-butyl-1,3-thiazolin-5-carbonsäure | Amt |
| 6-Amino-penicillansäure | Ape |
| α-Amino-pimelinsäure | Apm |
| α-Amino-korksäure | Asu |
| 2-Azetidin-carbonsäure | Aze |
| Aziridin-carbonsäure | Azi |
| Baikiain | Bai |
| C-Benzyl-phenylalanin (C,C-Bis-benzylglycin) | Bph |
| Canavanin | Can |
| Citrullin | Cit |
| 3,4-Dihydroxy-phenylalanin | Dpa |
| 2,2-Dimethyl-1,3-thiazolidin-carbonsäure | Dtc |
| Felinin | Fel |
| Isoserin | Ise |
| Isovalin | Iva |
| Kynurenin | Kyn |
| Leucenin | Lcn |
| Mimosin | Mim |

Minalin Min
Norleucin (α-Amino-capronsäure) Nle
Norvalin Nva
Oxalysin Oly
Pantonin (β,β-Dimethyl-homoserin) Pan
C-Phenyl-glycin Phg
β-Phenyl-serin Pse
Picolinsäure Pic
Pipecolinsäure Pec
(Pyrazolyl-3)-alanin Pza
(Pyridyl-2)-alanin Pya
Pyrrolidon-(5)-carbonsäure-(2) Pyr
Chinoxalin-2-carbonsäure Qin
Sarkosin Sar
Selono-cystein Sec
Seleno-methionin Sem
Tertiärleucin (Pseudoleucin) Tle
Thialysin Tly
1,3-Thiazolin-2-carbonsäure Tia
[1,3-Thiazolyl-(2)]-alanin Thi
Thyronin [3,5,3′-Trijod-0-(4-hydroxyphenyl)-tyrosin] Thy
Thyroxin (3,4,3′,5′-Tetrajod-thyronin) Thx

Schutzgruppen

Boc - tert.-Butoxycarbonyl
But - tert.-Butyl (als ätherbildende Gruppe)
OTmse - 2-(Trimethylsilyl)-äthoxy (als esterbildende Gruppe)
Z - Benzyloxycarbonyl (Carbobenzoxy)
Mtr - 4-Methoxy 2,3,6-trimethylbenzolsulfonyl
FMOC - 9-Fluorenyl-methyloxycarbonyl
Acm - Acetamidomethyl
DOD - 4,4′-Dimethoxydityl

Lösungsmittel

DMF - Dimethylformamid
THF - Tetrahydrofuran
DMA - Dimethylacetamid
IPE - Isopropyläther
TFA - Trifluoressigsäure
$CH_2Cl_2$ - Methylenchlorid
Sole - gesättigte Kochsalzlösung

Methoden

DC (TLC)-Dünnschichtchromatographie

In DC verwendet man, wenn nicht anders angegeben, Kieselgel als Adsorbens und beispielsweise folgende Systeme als Laufmittel:
Chloroform - Methanol - Essigsäure (85 : 10 : 5)
Chloroform - Methanol - Essigsäure (77,5 : 15 : 7.5)
Chloroform - Methanol - 32 %-ige Essigsäure (15 : 4 : 1)
Detektion: UV, Ninhydrin, Reindel-Hope-Reagens

Beispiele

Beispiel 1

60,85 g 3-Methoxy-4-formylphenol [R.C. Sheppard und B. Williams; Int. J. Pept. Prot. Res. 20, 451 (1982)] werden unter Rühren in einer Mischung von 16,0 g (0,4 mol) fester Natronlauge (NaOH), 1200 ml Wasser und 400 ml Methanol gelöst, wobei Sauerstoff auszuschliessen ist. Nun gibt man 15,13 g (0,4 mol) Natriumborhydrid ($NaBH_4$) so langsam in kleinen Portionen hinzu, dass die Innentemperatur nicht ansteigt. Der Reaktionsverlauf lässt sich dünnschichtchromatographisch verfolgen ($CHCl_3$-$CH_3OH$-$CH_3COOH$ 85 : 10 : 5 oder ähnliche Systeme; das Reaktionsprodukt 3-Methoxy-4-hydroxymethylphenol ist polarer als das Ausgangsprodukt). Der Aldehyd ist kurz nach Zugabe des Restes an Natriumborhydrid umgesetzt worden. Man lässt noch eine Stunde lang ausrühren. Dann gibt man 1 l Sole (gesättigte NaCl-Lösung) zu der leicht trüben Lösung und neutralisiert durch vorsichtige Zugabe von 57, 13 g (0,42 mol) Kaliumhydrogensulfat ($KHSO_4$) in kleinen Portionen. Da das Reaktionsprodukt 3-Methoxy-4-hydroxymethylphenol ausfällt, wird die Lösung immer trüber. Sie wird zweimal mit je 1500 ml Essigester extrahiert. Der Ph-Wert der wässrigen Phase, wird ständig kontrolliert und sollte immer noch bei 7 - 8 liegen.
Man gibt zu der organischen Phase ein wenig festes Natriumbicarbonat hinzu, um auch Spuren von Säuren zu binden. Die organische Phase wird dekantiert und zweimal mit 1 l Sole gewaschen. Man trocknet sie über Natriumsultat und engt im Vakuum bei ca. 20° C ein. Beim Einengen kann ein Teil des Produkts ausfallen. Der feste Rückstand kann sich auf der Oberfläche rötlich bis violett verfärben, falls noch Spuren von Säuren vorhanden sind. Allerdings haben solche Verfärbungen bei der Kupplung des erhaltenen Reaktionsproduktes 3-Methoxy-4-hydroxymethylphenol als Linker (Anker) an das im Beispiel 3 erwähnte Harz keine weitere Bedeutung. Diese Verfärbungen können auch auftreten, wenn der Rückstand noch im Vakuum getrocknet wird. Der trokkene Rückstand sollte zur weiteren Verwendung (Alkylierung) möglichst bald eingesetzt werden, wobei weitere Reinigungsoperationen nicht erforderlich sind.
Im Dünnschichtchromatogramm können weniger polare Verunreinigungen auftreten, die Oligomere des erhaltenen Phenols sein könnten. Verbindungen dieser Art lassen sich durch katalytische Mengen Säure leicht polymerisieren.
3-Methoxy-4-hydroxymethylphenol kann auch in kristalliner Form erhalten werden, wenn man den Eindampfrückstand in der gerade nötigen Menge Methanol-Essigester (1 : 1) löst und im Eisbad unter Rühren n-Hexan zutropft. Man lässt noch über Nacht im Tiefkühlschrank stehen, filtriert und erhält weisse Nadeln vom Schmp. 128 - 130° C.
Das kristalline 3-Methoxy-4-hydroxymethylphenol hat aber keine Vorteile gegenüber dem Rohprodukt bei seiner weiteren Verwendung erkennen lassen.

Beispiel 2

Man lässt 111,4 g (114,7 meq) mit 1 % Divinylbenzol vernetztes Chlormethylpolystyrol (Cl-Gehalt: 1,04 meq/g) in 750 ml Dimethylacetamid über Nacht langsam rühren. (Das verwendete DMA hat vor der Verwendung einige Tage über Molekularsiebe (4Å) gestanden). Zu dieser Suspension gibt man eine Lösung von 55,7 g (0,36 mol) 3-Methoxy-4-hydroxymethylphenol in 250 ml Dimethylacetamid. Die Mischung wird unter Rühren langsam auf 50° C erwärmt und 19,4 g Natriummethylat (0,366 mol) zugegeben. Das in Dimethylacetamid nicht lösliche Natriummethylat wird durch Phenolatbildung rasch verbraucht. Unter Ausschluss von Sauerstoff und Wasser rührt man noch weitere 7 Stunden bei 50° C weiter. Die erhaltene Suspension verfärbt sich langsam tiefrot.
Nach Abkühlen auf Raumtemperatur gibt man vorsichtig Wasser zu, um das abgeschiedene Natriumchlorid (Kochsalz) aufzulösen. Durch die Wasserzugabe wird das erhaltene Harz geschrumpft und fast völlig entfärbt. Man filtriert und verwirft das tiefrote Filtrat. Um das erhaltene Polymer p-(3-Methoxy-4-hydroxymethylphenoxymethyl)polystyrol zu quellen, wird dieses dreimal mit Dioxan oder Tetrahydrofuran (THF) gewaschen. Reste der Verfärbung werden durch dreimalige Behandlung mit Dioxan/Wasser (1 : 1) entfernt. Anschliessen kann noch mit anderen Lösungsmitteln gewaschen und getrocknet werden.
Das erhaltene Polymer (Harz) p-(3-Methoxy-4-hydroxymethylphenoxymethyl)polystyrol ist annähernd farblos oder leicht rosa oder beige verfärbt. Diese leichte Verfärbung lässt sich auch durch weiteres Waschen nicht mehr entfernen. Das erhaltene Polymer wird im Vakuum getrocknet und zeichnet sich durch einen Gehalt von weniger als 0,1 % Chlor aus.

Beispiel 3

Kupplung von 9-Fluorenlymethyloxycarbonyl-alanin (FMOC-Alanin) an das im Beispiel 2 erhaltene Harz:

a) Vor der Verwendung wird das erhaltene Harz p-(3-Methoxy-4-hydroxymethylphenoxymethyl)polystyrol mit einem Gemisch aus Dimethylformamid-Methylenchlorid [DMF/$CH_2Cl_2$ (1 : 4)] einige Male gewaschen

und vorgequollen (möglichst in dem gleichen Medium der nachfolgenden Reaktion), wobei unerwünschtes Wasser und Alkohol entfernt werden. 25,32 g (25,13 meq) p-(3-Methoxy-4-hydroxymethylphenoxymethyl)polystyrolharz werden, wie eingangs erwähnt, vorbehandelt und anschliessend bei 0° C in 100 ml eines Gemisches aus Dimethylformamid/Methylenchlorid(1 : 4) gerührt oder geschüttelt. Zu dieser Suspension werden 19,7 g FMOC-Alanin (50,43 mmol) und 230 mg 4-Dimethylaminopyridin (1,9 mmol) als Katalysator für die Veresterung zugegeben. Man rührt einige Minuten zur Aequilibrierung und fügt 10,74 g (52,1 mmol) Dicyclohexylcarbodiimid in kleinen Portionen zu. Die Suspension wird noch einige Stunden bei 0° C gerührt. Beim Rühren über Nacht erreicht der Ansatz dann langsam Raumtemperatur, da das für die Kühlung erforderliche Eis schmilzt. Nach 21 Stunden wird das durch Dicyclohexylharnstoff verunreinigte Polymer abfiltriert und auf der Fritte fünfmal mit einem Gemisch aus Dimethylformamid/Methylenchlorid gewaschen. Um den Dicyclohexylharnstoff zu entfernen, wird je dreimal mit Methylenchlorid/Isopropanol, Methylenchlorid, Methylenchlorid/Isopropanol und zweimal mit Isopropyläther gewaschen. Das gereinigte Polymer wird im Vakuum getrocknet.

FMOC-Gehalt: 0,685 meq FMOC-Alanin/g

Gravimetrische Bestimmung: 0,633 meq FMOC-Alanin/g

Racemisierungskontrolle: 0,1 % D-Alanin.

Es genügt, wenn man nur eine Probe für die FMOC-Bestimmung trocknet. Bei ausreichender Belandung muss das erhaltene Harz noch benzoyliert werden, ansonsten wird der Kupplungsvorgang nochmals wiederholt.

b) Hat man einen guten Umsatz bei der vorausgegangenen Kupplungsreaktion erzielt, wo müssen die im Polymerharz verbliebenen freien Hydroxygruppen blockiert werden. Für diesen Vorgang eignet sich z.B. Benzoylchlorid.

35,7 g des oben erhaltenen FMOC-Alanin-Harzes werden in 200 ml Methylenchlorid suspendiert. Unter Eiskühlung werden 18 ml (223 mmol) Pyridin und einige Minuten später 28 ml (215 mmol) Benzoylchlorid zugegeben. Das Polymer darf nicht mit freier Salzsäure in Berührung kommen. Man lässt dieses noch eine halbe Stunde im Reaktionsgemisch rühren und wäscht es mit Methylenchlorid, Dimethylformamid, Isopropanol, Isopropyläther und zweimal nochmals mit Methylenchlorid, Isopropanol und Isopropyläther. Nach dem Trocknen im Vakuum erhält man 37,8 g Harz, welches direkt für eine Peptidsynthese eingesetzt werden kann.

c) Das erhaltene Polymer wird wie folgt charakterisiert:

$C_1$) FMOC-Bestimmung:

Kleine eingewogene Proben werden 1/2 Stunde mit Piperidin/Dimethylformamid (1 : 4) behandelt, wodurch die FMOC-Gruppen abgespalten werden. Die Konzentration des erhaltenen Spaltproduktes 9-Fluorenylmethylpiperidin wird photometrisch bestimmt (bei 301 nm/$\epsilon_m$ 7200).

$C_2$) Gravimetrische Bestimmung (Abspaltung der FMOC-Aminosäure):

Eine eingewogene Probe wird mit 1 % Trifluoressigsäure ($TFA/CH_2Cl_2$) gespalten (s. Methode weiter unten). Das Harz wird gewaschen und getrocknet. Die Lösung der FMOC-Aminosäure wird eingeengt und nötigenfalls neutralisiert. Der erhaltene Rückstand wird getrocknet und zur gravimetrischen Bestimmung ausgewogen (ebenso das Harz).

$C_3$) Bestimmung des Stickstoffgehalts:

Es werden nur geringe Stickstoffwerte erwartet, sie liegen meistens unter 1 % (Ausnahmen: Arg, His, Lys, Orn). Durch Verunreinigungen (herrührend von den verwendeten Lösungsmitteln wie z.B. Dimethylformamid) werden unverhältnismässige Erhöhungen der erhaltenen Stickstoffwerte erhalten.

Die zuverlässigste und schnellste Methode zur Bestimmung der Beladung ist die FMOC-Bestimmung. Nach $C_2$) kann man auch die Spaltungsbedingungen testen.

$C_4$) Kontrolle der Racemisierung:

An einer eingewogenen Probe werden zunächst mit Piperidin/DMF (1 : 4) die FMOC-Gruppen abgespalten. Diese wird sorgfältig ausgewaschen und mit einem Lösungsgemisch $TFA/CH_2Cl_2$ = 1 : 1 (v/v) gespalten. Diese Spaltung muss nicht quantitativ verlaufen. Das erhaltene Polymer wird abfiltriert und gut mit Methylenchlorid und n-Propanol ausgewaschen. Das Filtrat wird einige Male unter Zusatz von n-Propanol vollständig eingedampft. Die optische Reinheit des N-Trifluoracetyl-n-propylesters der Aminosäure wird gaschromatographisch an einer chiralen Säule überprüft.

Beispiel 4

a) Die Synthese an dem acylierten Polymer (nach Beispiel 2 erhalten) unterscheidet sich nicht von der in vielen Publikationen dokumentierten Festphasen-Peptidsynthese (SPPS) mit FMOC-Aminosäuren. Diese Methode ist z.B. bei J. Meierhofer et al., Int. f. Pept. Prot. Res. 13, 35 (1979) beschrieben. Diese wird

durch den Kaiser-Test (E. Kaiser et al., Anal. Biochem. 34, 595 (1970)) und durch die FMOC-Bestimmung (nach $c_1$) quantitativ verfolgt.
Als Reaktionsmedien werden DMF und DMF/$CH_2Cl_2$ (Methylenchlorid) eingesetzt.
Die FMOC-Aminosäuren werden in einem 2-fachen (oder höheren) Ueberschuss durch Aktivierung mit Dicyclohexylcarbodiimid/4-Dimethylaminopyridin gekuppelt.
b) Abspaltung eines vollgeschützten Peptides vom Harz (Peptidharz)
Vor der Spaltung muss das Harz sorgfältig mit Methylenchlorid vorgewaschen oder einige Stunden in einer Methylenchloridlösung gerührt werden. Verunreinigungen wie Wasser oder Aethylalkohol stören bei der Spaltung. Dicyclohexylharnstoffreste (DCU) werden bei der Spaltung aufgelöst, da diese eine Salz mit der Trifluoressigsäure (TFA) bilden und damit ein Teil der verwendeten Säure abfangen und hiermit das Peptid verunreinigen könnten.
c) Kassinin 1 - 5
(ein Peptid, das keine Löslichkeitsprobleme verursacht) Boc-Asp (OBut)-Val-Pro-Lys(Boc)-Ser(But)OH
12,63 g Peptidharz werden 5 Stunden lang in 100 ml Methylenchlorid gerührt. Bei Zugabe von 100 ml eines 2 %-igen TFA/$CH_2Cl_2$-Gemisches wird das Harz leicht rosa verfärbt. Man rührt dieses Gemenge eine halbe Stunde und filtriert. Das Harz wird erneut in 200 l eines 1 %-igen TFA/$CH_2Cl_2$-Gemisches suspendiert und anschliessend noch eine dreiviertel Stunde lang gerührt, wobei sich das Harz violett verfärbt. Nach Filtration wird das Harz noch ein drittes Mal mit 200 ml eines 1- %-igen TFA/$CH_2Cl_2$-Gemisches 1/2 Stunde lang behandelt, filtriert und ausgewaschen.
Die drei nacheinander ausgeführten Spaltungen werden mittels DC verfolgt. Der Hauptteil des Peptids fällt beim Zweiten Spaltungsvorgang an. Beim dritten Spaltungsvorgang wird nur noch wenig Peptid erhalten, so dass auf eine weitere Säurebehandlung verzichtet wird. Im Anschluss an diese Spaltungsreaktionen wird das Polymer mit Methylenchlorid und Essigester ausgewaschen. Beim Waschen mit Essigester verschwindet seine intensive Farbe umgehend.
Die gesammelten Filtrate der Spaltungen werden sofort neutralisiert. Die vereinigten Filtrate werden eingeengt, wobei das abdestillierte Methylenchlorid durch Essigester ersetzt wird.
Diese Lösung wird mit 150 ml eines 5 %-igen Lösungsgemisches aus Natriumhydrogencarbonat/Sole (2 : 1) gewaschen, die beiden Phasen trennen sich langsam. Die erhaltene obere Phase wäscht man mit 150 ml einer 1n Kaliumhydrogensulfat/Sole (2 : 1)-Lösung und danach mit 100 ml einer 10 %-igen Kochsalzlösung. Verbliebene Säurespuren werden durch zweimaliges Waschen mit 100 ml Sole entfernt. Die erhaltene organische Phase wird über Natriumsulfat getrocknet und zur Trokkne eingeengt. Der feste Rückstand konnte nicht durch weiteres Auflösen und Fällen mit IPE weiter gereinigt werden. DC: ein Fleck mit einer geringen polaren Verunreinigung.

| Elementaranalyse: | | | |
|---|---|---|---|
| | C | H | N |
| berechnet | 57,37 % | 8,58 % | 9,80 % |
| gefunden | 57,06 % | 8,51 % | 9,38 % |

In analoger Weise können auch längere Peptide nach der beschriebenen Methode aufgearbeitet werden.

Beispiel 5

ANF 1 - 10
Boc-Ser(But)-Lei-Arg(Mtr)-Arg(Mtr)-Ser(But)-Ser(But)-Cys(Acm)-Phe-Gly-Gly-OH

5,03 g Peptidylharz werden mehrmals mit Methylenchlorid gewaschen. Anschliessen wird das Harz vier Mal mit 100 ml einer Lösung enthaltend 1 % TFA in $CH_2Cl_2$ eine halbe Stunde unter Rühren behandelt. Bei der ersten Spaltung werden 0,2 ml m-Kresol zugefügt. Das Harz verfärbt sich rosa. Diese Verfärbung vertieft sich auch bei den späteren Spaltungen nicht. Diese Spaltungsreaktionen werden dünnschichtchromatographisch verfolgt. Der grösste Teil des Peptides wird während der ersten und zweiten (Spaltung) Säurebehandlung abgespalten. Bei der vierten Säurebehandlung tritt nur noch eine Spur Peptid auf. Das Harz wird mit Methylenchlorid gewaschen und anschliessend getrocknet. Die erhaltenen Filtrate werden mit Pyridin neutralisiert. Ein schneller Viskositätsanstieg ist die Folge; die Filtrate gelieren, schliesslich fällt eine amorphe Masse aus. Ein Teil des Methylenchlorids wird entfernt. Das verbleibende Oel wird unter kräftigem

Rühren in Isopropyläther (IPE) suspendiert. Um Pyridin und Pyridiniumsalze zu entfernen, wird diese Suspension mit verdünnter Salzsäure (ph~3) verrührt. Das zwischen den Phasen suspendierte Peptid wird abfiltriert und mit Wasser und Isopropyläther gewaschen.

Eine derartige Sequenz konnte bisher weder durch konventionelle Lösungssynthesen, noch mit den bisher bekannten Festphasensynthesen (SPPS) hergestellt werden.

Ausbeite: Polymer 2,90 g

(Gewichtsverlust 2,13 g (42,3 %)), Peptid 2,43 g (enthält noch Pyridiniumsalze, kann aber nach einer weiteren Säurebehandlung zur Kupplung eingesetzt werden).

Die theor. Ausbeute (Peptid) beträgt 2,89 g unter der Voraussetzung, dass FMOC-Gly mit einer Ausbeute von 84 % ans Harz gekuppelt wird und die nachfolgenden Kupplungen annähernd vollständig (quantitativ) verliefen. Es resultiert ein Peptidharz, das sich aus 42,5 % Polymer und 57,5 % Peptid zusammensetzt. Legt man den FMOC-Gehalt nach Ankupplung der vorletzten Aminosäure (Leu) zugrunde, sollte das Peptidylharz aus 42,8 % Polymer und 57.2 % Peptid bestehen (bzw. 0,331 meq ANF 1-10/g Harz).

Durch einen weiteren Spaltungsversuch nach Quellen und Schrumpfen des Polymeren wird kein weiteres Peptid erhalten, auch nicht durch Behandlung mit einer 2 %-igen $TFA/CH_2Cl_2$-Lösung.

Setzt man bei der Spaltung einen "scavenger" wie z.B. m-Kresol ein, wird sich das Polymere nicht violett verfärben, sondern nur leicht rosa.

## Patentansprüche

**1.** Trägerharze, bestehend aus einem Polymerenharz der Formel I

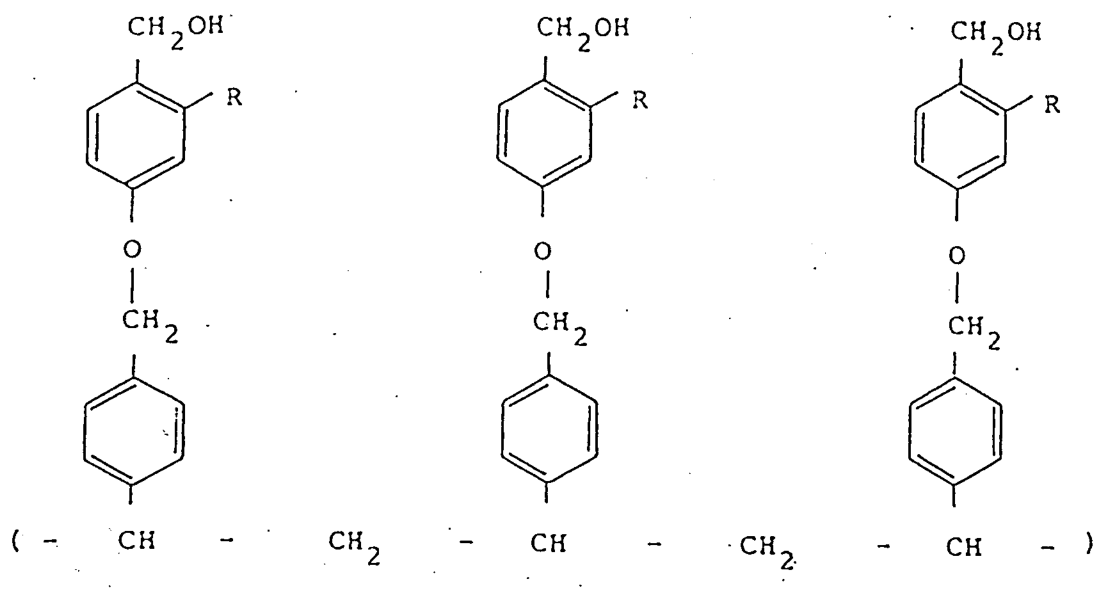

(I)

in welcher R eine Alkoxy-, Alkylthio- oder eine

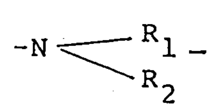

Gruppe bedeutet, in welcher $R_1$ und $R_2$ bis mit 7, vorzugsweise bis mit und mit 4 Kohlenstoffatome enthalten, welches mit 0,5 - 2 % eines gegebenenfalls durch Halogen oder auch Alkyl substituierten Divinylbenzols vernetzt ist.

**2.** Neue Trägerharze gemäss Anspruch 1, bestehend aus einem Polymeren der Formel I, in der R bis mit 7, vorzugsweise bis mit und mit 4 Kohlenstoffatome enthalten, welches mit 1 - 2 % des unsubstituierten 1,4-Divinylbenzols vernetzt ist.

**3.** Trägerharze gemäss Ansprüchen 1 und 2, bestehend aus einem Polymeren der Formel I, in der R Methoxy bedeutet, welches mit 1 - 2 % des unsubstituierten 1,4-Divinylbenzols vernetzt ist.

**4.** Verfahren zur Herstellung von Polymeren der Formel I, in welcher R die im Anspruch 1 angegebenen Bedeutungen hat, dadurch gekennzeichnet, dass man Chlormethylpolystyrolharze der Formel II,

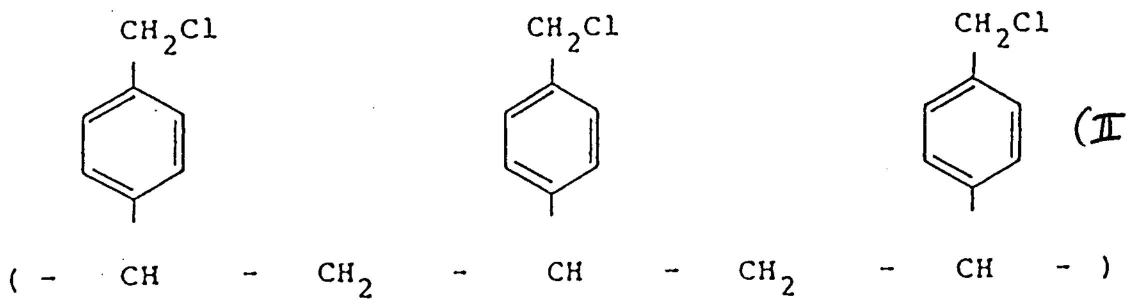

(II)

welche mit 0,5 - 2 % eines gegebenenfalls durch Halogen oder bis mit 7, vorzugsweise bis mit und mit 4 Kohlenstoffatome enthaltenden Divinylbenzols vernetzt sind, unter basischen Bedingungen mit einem 4-Hydroxymethyl-3-R-subst.-phenol der Formel III

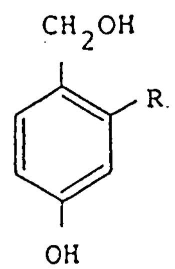

(III)

veräthert.

**5.** Verfahren zur Herstellung von Polymeren der Formel I gemäss Anspruch 4 dadurch gekennzeichnet, dass man die Verätherung der phenolischen OH-Gruppe in Gegenwart von Natriummethylat oder -äthylat ausführt.

**6.** 4-Hydroxymethyl-3-R-subst.-phenole der Formel III

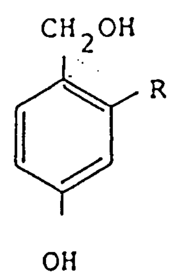

(III)

in welcher R die im Anspruch 1 angegebene Bedeutung hat.

**7.** 4-Hydroxymethyl-3-methoxy-phenol.

**8.** Verfahren zur Herstellung von 4-Hydroxymethyl-3-R-subst.-phenolen der Formel III, dadurch gekennzeichnet, dass man 3-R-subst.-4-formylphenole reduziert.

**9.** Fragmente (Peptidylharze) bestehend aus einem Polymeren der Formel IV

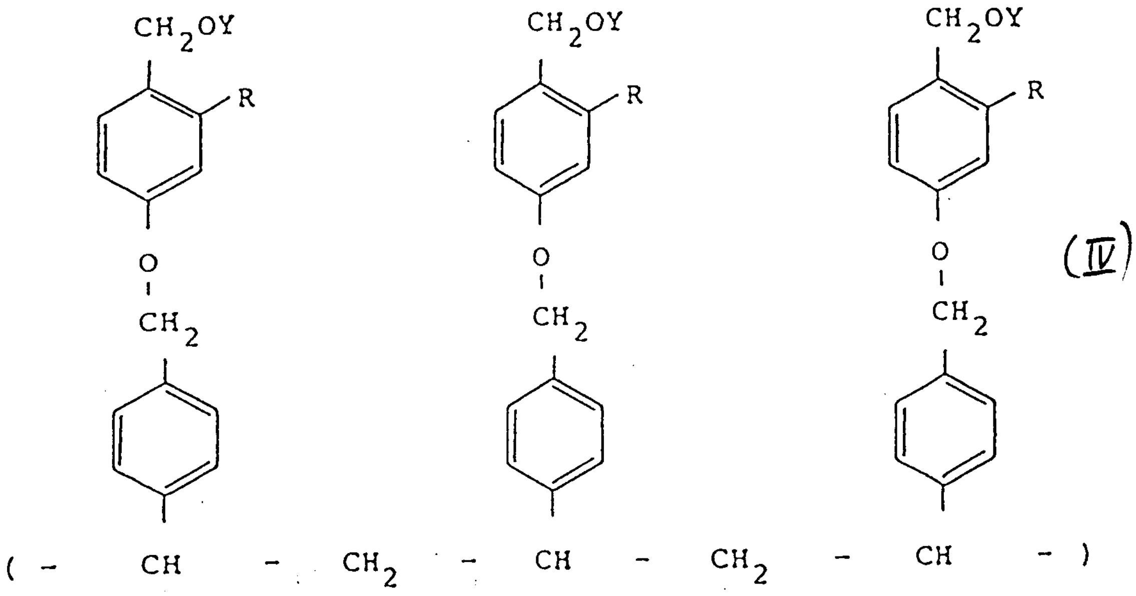

(IV)

welche mit 0,5 - 2 % eines gegebenenfalls durch Halogen oder bis mit 7, vorzugsweise bis mit und mit 4 Kohlenstoffatomen substituierten Divinylbenzols vernetzt sind, und in welchen R die unter der Formel I angegebene Bedeutung hat und Y den Rest einer Aminosäure bedeutet, welche mit der endständigen Carboxylgruppe an das Trägerharz kondensiert ist, in welcher die funktionellen Gruppen der Aminosäure gegebenenfalls durch Schutzgruppen geschützt sind.

**10.** Fragmente bestehend aus einem Polymeren der Formel IV gemäss Anspruch 9, in welchem R eine Alkoxygruppe bedeutet und Y den Rest einer als Peptidbaustein natürlich vorkommenden $\alpha$-Aminosäure der L-Reihe oder deren nahe verwandte Analoge wie insbesondere der Enantiomeren der unnatürlichen D-Reihe, in welcher die funktonellen Gruppen der Aminosäure gegebenenfalls durch Schutzgruppen geschützt sind.

**11.** Fragmente bestehend aus einem Polymeren der Formel IV gemäss den Ansprüchen 9 und 10, in welchem R Methoxy bedeutet und Y den Rest einer natürlich vorkommenden $\alpha$-Aminosäure bedeutet, in welcher die funktionellen Gruppen der Aminosäure gegebenenfalls durch Schutzgruppen geschützt sind.

**12.** Ein Fragment bestehend aus einem Polymeren der Formel IV gemäss den Ansprüchen 9 - 11, in welchem R Methoxy und Y einen Alanin-Rest bedeutet.

**13.** Ein Fragment bestehend aus einem Polymeren der Formel IV gemäss den Ansprüchen 9 - 11, in welchem R Methoxy und Y einen Arginin-Rest bedeutet.

**14.** Ein Fragment bestehend aus einem Polymeren der Formel IV gemäss den Ansprüchen 9 - 11, in welchem R Methoxy und Y einen Asparagin-Rest bedeutet.

**15.** Ein Fragment bestehend aus einem Polymeren der Formel IV gemäss den Ansprüchen 9 - 11, in welchem R Methoxy und Y den Asparaginsäure-Rest bedeutet.

**16.** Ein Fragment bestehend aus einem Polymeren der Formel IV gemäss den Ansprüchen 9 - 11, in welchem R Methoxy und Y den Glutamin-Rest bedeutet.

**17.** Ein Fragment bestehend aus einem Polymeren der Formel IV gemäss den Ansprüchen 9 - 11, in welchem R Methoxy und Y den Glutaminsäure-Rest bedeutet.

**18.** Ein Fragment bestehend aus einem Polymeren der Formel IV gemäss den Ansprüchen 9 - 11, in welchem R Methoxy und Y den Glycin-Rest bedeutet.

**19.** Ein Fragment bestehend aus einem Polymeren der Formel IV gemäss den Ansprüchen 9 - 11, in welchem R Methoxy und Y den Histidin-Rest bedeutet.

**20.** Ein Fragment bestehend aus einem Polymeren der Formel IV gemäss den Ansprüchen 9 - 11, in welchem R Methoxy und Y den Isoleucin-Rest bedeutet.

**21.** Ein Fragment bestehend aus einem Polymeren der Formel IV gemäss den Ansprüchen 9 - 11, in welchem R Methoxy und Y den Leucin-Rest bedeutet.

**22.** Ein Fragment bestehend aus einem Polymeren der Formel IV gemäss den Ansprüchen 9 - 11, in welchem R Methoxy und Y den Lysin-Rest bedeutet.

**23.** Ein Fragment bestehend aus einem Polymeren der Formel IV gemäss den Ansprüchen 9 - 11, in welchem R Methoxy und Y den Methionin-Rest bedeutet.

**24.** Ein Fragment bestehend aus einem Polymeren der Formel IV gemäss den Ansprüchen 9 - 11, in welchem R Methoxy und Y den Phenylalanin-Rest bedeutet.

**25.** Ein Fragment bestehend aus einem Polymeren der Formel IV gemäss den Ansprüchen 9 - 11, in welchem R Methoxy und Y den Prolin-Rest bedeutet.

**26.** Ein Fragment bestehend aus einem Polymeren der Formel IV gemäss den Ansprüchen 9 - 11, in welchem R Methoxy und Y den Serin-Rest bedeutet.

**27.** Ein Fragment bestehend aus einem Polymeren der Formel IV gemäss den Ansprüchen 9 - 11, in welchem R Methoxy und Y den Threonin-Rest bedeutet.

**28.** Ein Fragment bestehend aus einem Polymeren der Formel IV gemäss den Ansprüchen 9 - 11, in welchem R Methoxy und Y den Tryptophan-Rest bedeutet.

**29.** Ein Fragment bestehend aus einem Polymeren der Formel IV gemäss den Ansprüchen 9 - 11, in welchem R Methoxy und Y den Tyrosin-Rest bedeutet.

**30.** Ein Fragment bestehend aus einem Polymeren der Formel IV gemäss den Ansprüchen 9 - 11, in welchem R Methoxy und Y den Valin-Rest bedeutet.

**31.** Fragmente bestehend aus einem Polymeren der Formel IV gemäss den Ansprüchen 12 - 30, in welchem die funktionellen Gruppen der α-Aminosäurereste Y durch Schutzgruppen geschützt sind.

**32.** Fragmente bestehend aus einem Polymeren der Formel IV gemäss den Ansprüchen 12 - 30, in welchem die α-Aminogruppen der α-Aminosäurereste Y durch die Fluorenylmethyloxycarbonyl-Schutzgruppe geschützt sind.

**33.** Verfahren zur Herstellung eines Fragments bestehend aus einem Polymeren der Formel IV, dadurch gekennzeichnet, dass man ein Trägerharz, bestehend aus einem Polymerenharz der Formel I,

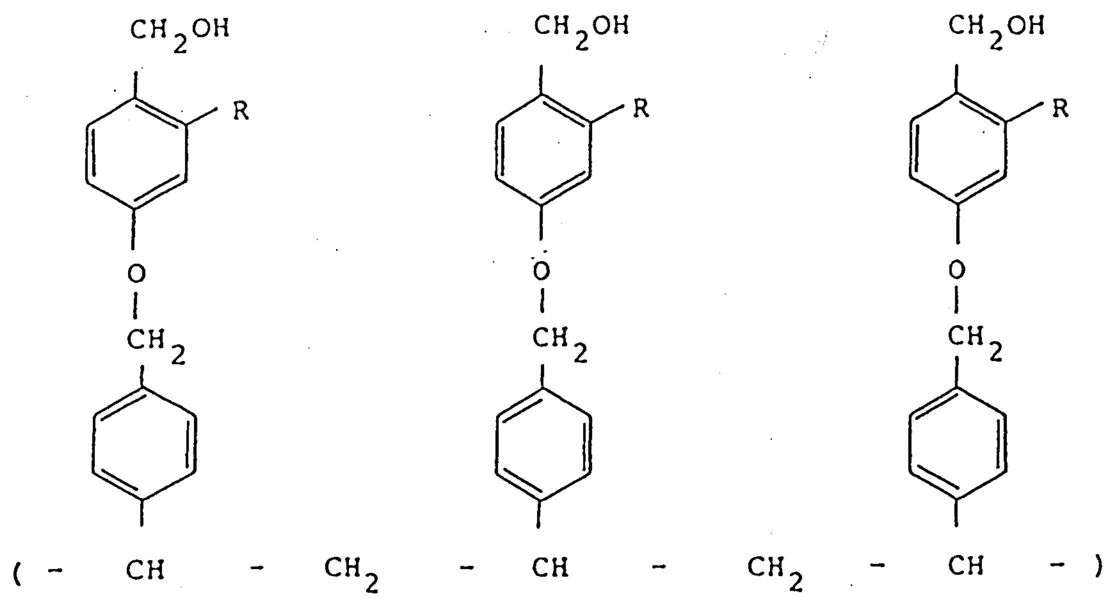

in welcher R eine Alkoxy-, Alkylthio- oder eine

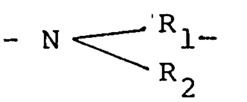

Gruppe bedeutet, in welcher $R_1$ und $R_2$ bis mit 7, vorzugsweise bis mit und mit 4 Kohlenstoffe enthalten, welches mit 0,5 - 2 % eines gegebenenfalls durch Halogen oder auch Alkyl substituierten Divinylbenzols vernetzt ist, mit einer Aminosäure, in der die α-Aminogruppe und andere funktionelle Gruppen gegebenenfalls durch Schutzgruppen geschützt sind, über die Carboxylgruppe durch Veresterung kondensiert.

**34.** Verfahren gemäss Anspruch 33, dadurch gekennzeichnet, dass man die Veresterung durch Aktivierung der Säurefunktion ausführt.

**35.** Verwendung der gemäss Ansprüchen 1 - 3 beanspruchten Trägerharze zur Peptidsynthese.

**36.** Verwendung der gemäss Ansprüchen 9 - 32 beanspruchten Fragmente zur Peptidsynthese.

## Claims

**1.** Resin-linker compounds consisting of a polymeric resin of formula I

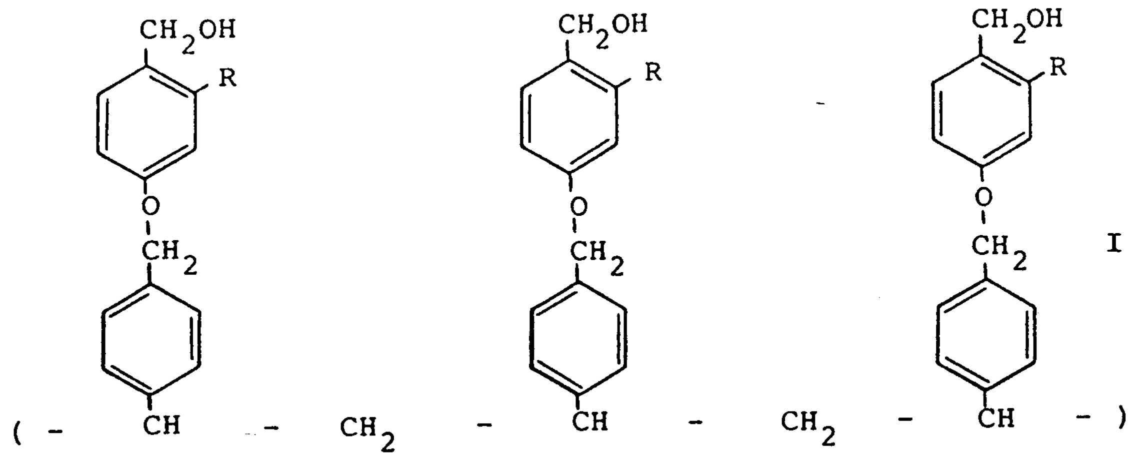

I

in which R represents an alkoxy-, alkylthio- or a

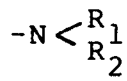

group in which $R_1$ and $R_2$ contain up to seven, preferentially up to four carbon atoms. The polymeric compound is optionally cross-linked with 0,5 - 2 % of divinylbenzene optionally substituted by halogen or alkyl.

2. Resin-linker compounds according to claim 1, consisting of a polymer of formula I in which the groups R contain up to seven, preferentially up to four carbon atoms. The polymer is cross-linked with 1 - 2 % unsubstituted 1,4-divinylbenzene.

3. Resin-linker compounds according to claims 1 and 2, consisting of a polymer of formula I in which R represents methoxy, the polymer is cross-linked with 1 - 2 % unsubstituted 1,4-divinylbenzene.

4. Process for the preparation of polymers of formula I where R represents the groups specified in claim 1, characterized by reacting chloromethylpolystyrene resin of formula II,

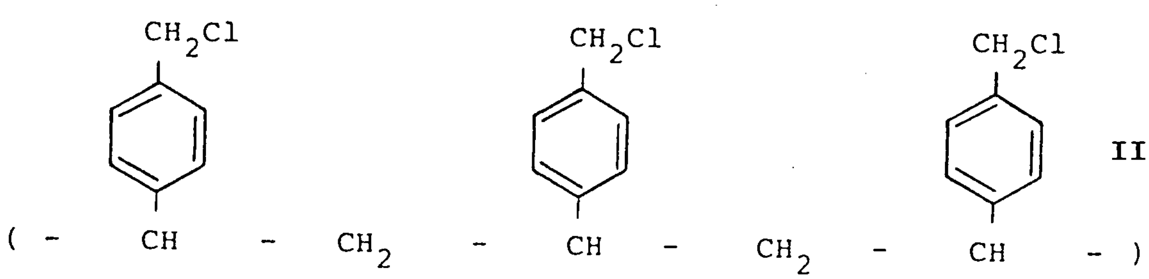

II

cross-linked with 0,5 - 2 % of optionally substituted (by halogen or by alkyl containing up to seven, preferentially up to four carbon atoms) divinylbenzene. Reaction is carried out under basic conditions with a 4-hydroxymethyl-3-R-substituted phenol of formula III and represents an ether formation (etherification)

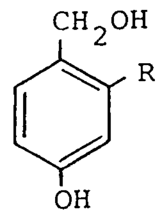

III

5. Process for the preparation of polymers of formula I according to claim 4 characterized by etherification of the phenolic hydroxyl group in the presence of sodium methoxide or ethoxide.

6. 4-hydroxymethyl-3-R-substituted phenols of formula III in which R represents the groups mentioned in claim 1.

7. 4-hydroxymethyl-3-methoxyphenol

8. Process for the preparation of 4-hydroxymethyl-3-R-substituted phenols characterized by reduction of the corresponding 3-R-substituted-4-formylphenols.

9. Fragments (peptidoylresins) consisting of a polymer of formula IV,

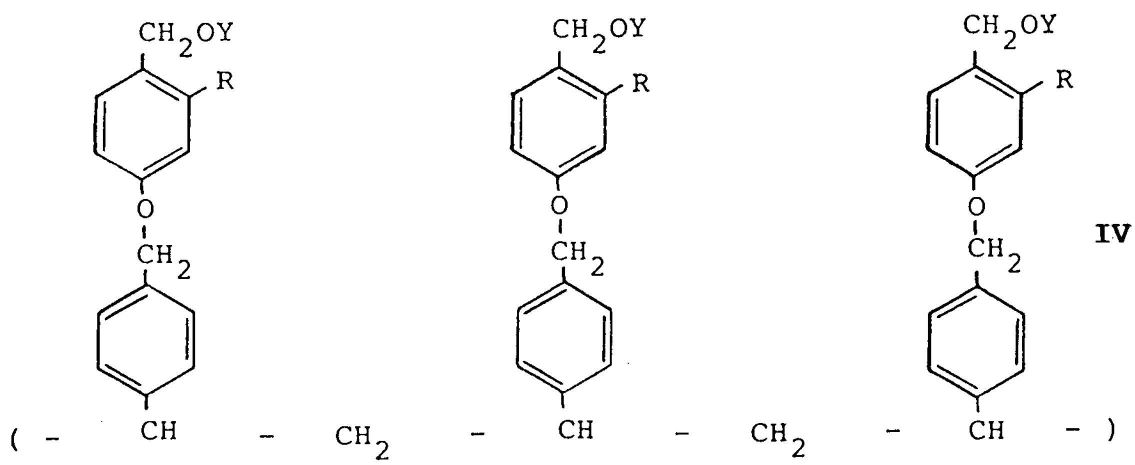

IV

optionally cross-linked with 0,5 - 2 % of an optionally substituted (by halogen or alkyl containing up to four carbon atoms) divinylbenzene. R represents the groups mentioned under formula I in claim 1, Y stands for the amino acid that represents the carboxy terminus of the peptide to be made. It is directly attached to the resin; other functional groups - if any - of this amino acid may be blocked by protecting groups.

10. Fragments consisting of a polymer of formula IV according to claim 9, in which R represents an alkoxy group and Y the residue of an amino acid, naturally occuring and belonging to the L-series, or a closely related analog, such as enantiomeric forms belonging to the unnatural D-series, in which the functional group(s) is (are) optionally protected.

11. Fragments consisting of a polymer of formula IV according to claims 9 and 10, in which R represents a methoxy group and Y the residue of a naturally occuring $\alpha$-amino acid in which functional groups are optionally blocked by protecting groups.

12. A fragment consisting of a polymer of formula IV according to claims 9 to 11, in which R is equal to methoxy and Y represents an alanine residue.

13. A fragment consisting of a polymer of formula IV according to claims 9 to 11, in which R is equal to

methoxy and Y represents an arginine residue.

**14.** A fragment consisting of a polymer of formula IV according to claims 9 to 11, in which R is equal to methoxy and Y represents an asparagine residue.

**15.** A fragment consisting of a polymer of formula IV according to claims 9 to 11, in which R is equal to methoxy and Y represents an aspartic acid residue.

**16.** A fragment consisting of a polymer of formula IV according to claims 9 to 11, in which R is equal to methoxy and Y represents a glutamine residue.

**17.** A fragment consisting of a polymer of formula IV according to claims 9 to 11, in which R is equal to methoxy and Y represents a glutamic acid residue.

**18.** A fragment consisting of a polymer of formula IV according to claims 9 to 11, in which R is equal to methoxy and Y represents a glycine residue.

**19.** A fragment consisting of a polymer of formula IV according to claims 9 to 11, in which R is equal to methoxy and Y represents a histidine residue.

**20.** A fragment consisting of a polymer of formula IV according to claims 9 to 11, in which R is equal to methoxy and Y represents an isoleucine residue.

**21.** A fragment consisting of a polymer of formula IV according to claims 9 to 11, in which R is equal to methoxy and Y represents a leucine residue.

**22.** A fragment consisting of a polymer of formula IV according to claims 9 to 11, in which R is equal to methoxy and Y represents a lysine residue.

**23.** A fragment consisting of a polymer of formula IV according to claims 9 to 11, in which R is equal to methoxy and Y represents a methionine residue.

**24.** A fragment consisting of a polymer of formula IV according to claims 9 to 11, in which R is equal to methoxy and Y represents a phenylalanine residue.

**25.** A fragment consisting of a polymer of formula IV according to claims 9 to 11, in which R is equal to methoxy and Y represents a proline residue.

**26.** A fragment consisting of a polymer of formula IV according to claims 9 to 11, in which R is equal to methoxy and Y represents a serine residue.

**27.** A fragment consisting of a polymer of formula IV according to claims 9 to 11, in which R is equal to methoxy and Y represents a threonine residue.

**28.** A fragment consisting of a polymer of formula IV according to claims 9 to 11, in which R is equal to methoxy and Y represents a tryptophan residue.

**29.** A fragment consisting of a polymer of formula IV according to claims 9 to 11, in which R is equal to methoxy and Y represents a tyrosine residue.

**30.** A fragment consisting of a polymer of formula IV according to claims 9 to 11, in which R is equal to methoxy and Y represents a valine residue.

**31.** Fragments consisting of a polymer of formula IV according to claims 12 to 30, in which the functional groups of the $\alpha$-amino acid residues Y are blocked by protecting groups.

**32.** Fragments consisting of a polymer of formula IV according to claims 12 to 30, in which the $\alpha$-amino groups of the $\alpha$-amino acid residues Y are blocked by the fluorenylmethyloxycarbonyl protecting group.

**33.** Process for the manufacture of a fragment consisting of a polymer of formula IV characterized by esterification of a carrier resin composed of a polymeric resin of formula I,

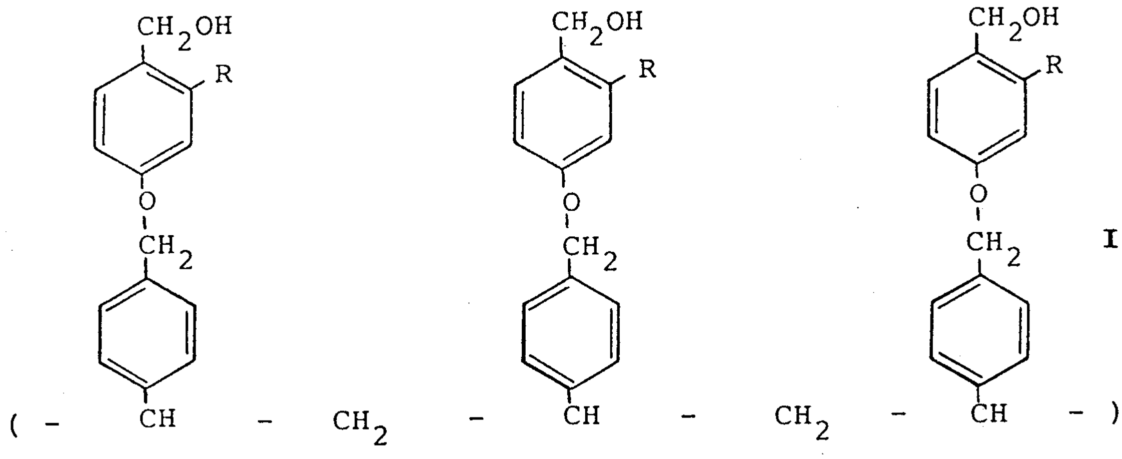

I

in which R represents an alkoxy-, alkylthio- or a

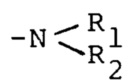

group, with $R_1$ and $R_2$ standing for an alkyl residue containing up to seven, preferentially up to four carbon atoms. Formula I may be cross-linked with 0,5 - 2 % of a divinylbenzene that is optionally substituted by halogen or alkyl. The esterifying carboxyl component consists of an amino acid whose $\alpha$-amino group and other functional groups - if any - are blocked by protecting groups and whose carboxyl group is used in the esterification.

**34.** Process according to claim 33, characterized by achieving esterification through activation of the carboxyl function.

**35.** Use of the described carrier-resin combination according to claims 1 to 3 for the synthesis of peptides.

**36.** Use of the fragments claimed in claims 9 to 32 for the synthesis of peptides.

**Revendications**

**1.** Des combinaisons résine-linker se composant d'une résine polymérique selon la formule générale I,

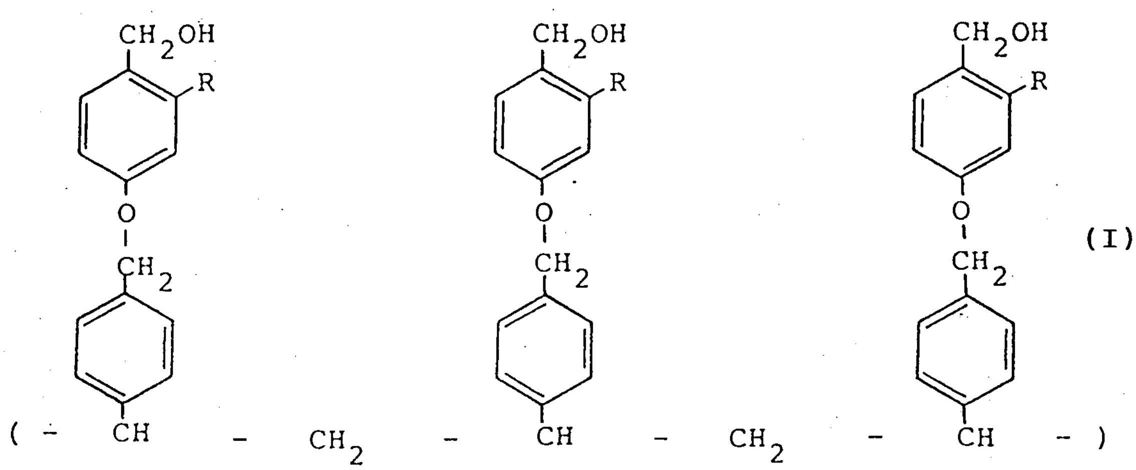

(I)

dans laquelle R représente soit un groupe alkoxy, soit un groupe alkylthio, soit le groupe

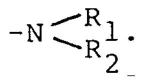

$R_1$ et $R_2$ peuvent contenir jusqu'à sept atomes de carbone, de préférence jusqu'à quatre atomes de carbone. Ce composé polymérique est lié transversalement avec, au choix, 0.5 à 2 % de divinylbenzène substitué soit par un halogène soit par un groupe alkyle.

**2.** Des combinaisons résine-linker selon la demande de brevet 1, se composant du polymère de la formule générale I, dans laquelle les groupes R contiennent jusqu'à sept atomes de carbone, de préférence jusqu'à quatre atomes de carbone. Ce polymère est lié transversalement avec 1 - 2 % de 1.4-divinylbenzène non substitué.

**3.** Des combinaisons résine-linker selon les demandes de brevet 1 et 2, se composant du polymère de la formule générale I, dans laquelle R représente le groupe méthoxy. Ce polymère est lié transversalement avec 1 - 2 % de 1.4-divinylbenzène non substitué.

**4.** Procédé de préparation des polymères de la formule générale I, dans laquelle R représente les groupes cités dans la demande de brevet 1, caractérisé par la réaction d'une résine polystyrène chlorométhylé selon la formule générale II.

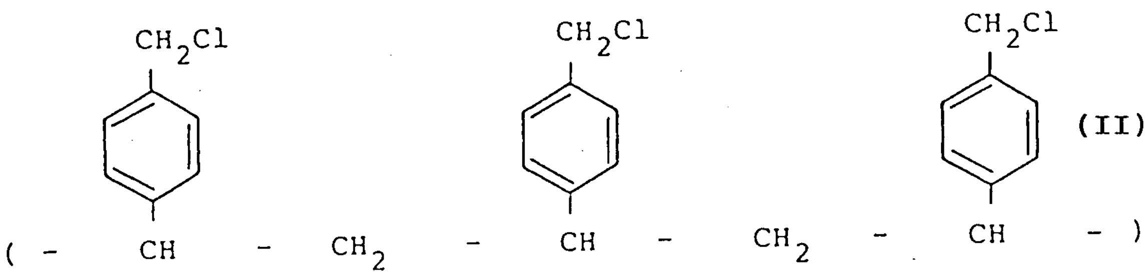

(II)

Ce polymère est lié transversalement (cross-linked) avec, au choix, 0.5 à 2 % de divinylbenzène substitué soit par un halogène soit par un groupe alkyle contenant jusqu'à sept atomes de carbone, de préférence jusqu'à quatre atomes de carbone. La réaction est effectuée en milieu basique avec un 4-hydroxyméthyl- 3-R-phénol selon la formule générale III et représente une formation d'éther.

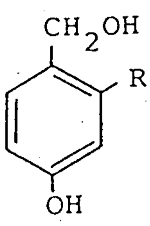

(III)

**5.** Procédé de préparation des polymères de la formule générale I selon la demande de brevet 4, caractérisé par étherification d'un groupe hydroxyle du phénol en presence de méthoxide de sodium ou d'éthoxide de sodium.

**6.** Des 4-hydroxyméthyl-3-R-phénols de la formule générale III, dans laquelle R représente les groupes mentionnés à la demande de brevet 1.

**7.** 4-hydroxyméthyl-3-méthoxyphénol.

8. Procédé de préparation des 4-hydroxyméthyl-3-R-phénols caractérisé par la réduction des 3-R-substitués-4-formylphénols correspondants.

9. Fragments (peptidoylrésines) se composant d'un polymère de la formule générale IV,

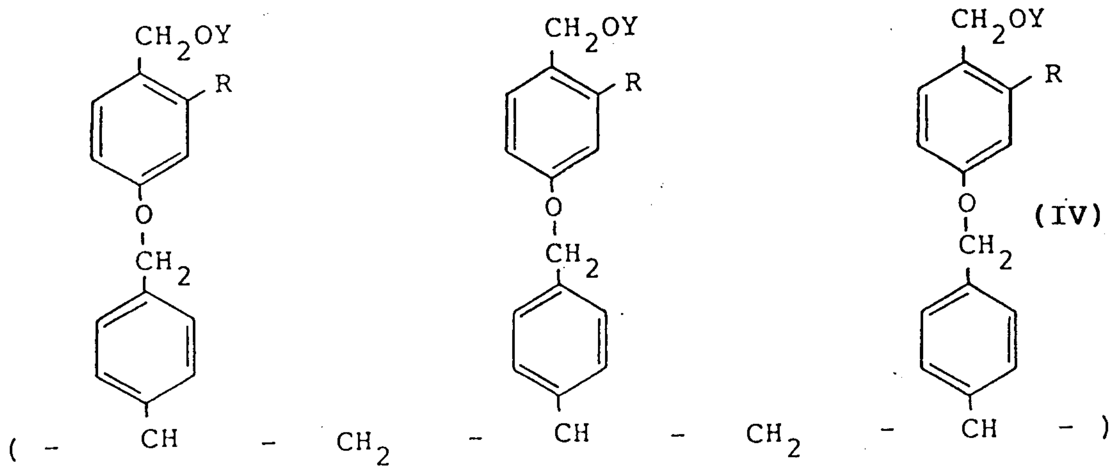

(IV)

lié transversalement avec, au choix, 0.5 à 2 % de divinylbenzène substitué soit par un halogène soit par un groupe alkyle contenant jusqu'à quatre atomes de carbone. R représente les groupes mentionnés à la demande de brevet 1, Y correspond à l'acide aminé de l'extrémité carboxylique (Ct) du peptide synthétisé. Cet acide aminé est directement lié par son extrémité carboxylique à la résine. Les autres groupes fonctionnels de cet acide aminé, s'il y en a, sont bloqués par des groupes protecteurs.

10. Fragments se composant d'un polymère selon la formule générale IV selon la demande de brevet 9. R y représente un groupe alkoxy et Y y représente soit le reste d'un acide aminé naturel (série L), soit un acide aminé étroitement analogue, tel qu'un énantiomère (série D), dans lequel le(s) groupe(s) fonctionnel(s) est (sont) facultativement protégé(s).

11. Fragments se composant d'un polymère selon la formule générale IV selon les demandes de brevet 9 et 10. R y représente un groupe méthoxy et Y le reste d'un acide aminé naturel, dans lequel les groupes fonctionnels sont facultativement protégés.

12. Un fragment se composant d'un polymère selon la formule générale IV selon les demandes de brevet 9 à 11, dans lequel R correspond au groupe méthoxy et Y représente le reste d'alanine.

13. Un fragment se composant d'un polymère selon la formule générale IV selon les demandes de brevet 9 à 11, dans lequel R correspond au groupe méthoxy et Y représente le reste d'arginine.

14. Un fragment se composant d'un polymère selon la formule générale IV selon les demandes de brevet 9 à 11, dans lequel R correspond au groupe méthoxy et Y représente le reste d'asparagine.

15. Un fragment se composant d'un polymère selon la formule générale IV selon les demandes de brevet 9 à 11, dans lequel R correspond au groupe méthoxy et Y représente le reste de l'acide aspartique.

16. Un fragment se composant d'un polymère selon la formule générale IV selon les demandes de brevet 9 à 11, dans lequel R correspond au groupe méthoxy et Y représente le reste de glutamine.

17. Un fragment se composant d'un polymère selon la formule générale IV selon les demandes de brevet 9 à 11, dans lequel R correspond au groupe méthoxy et Y représente le reste de l'acide glutamique.

18. Un fragment se composant d'un polymère selon la formule générale IV selon les demandes de brevet 9 à 11, dans lequel R correspond au groupe méthoxy et Y représente le reste de glycine.

**19.** Un fragment se composant d'un polymère selon la formule générale IV selon les demandes de brevet 9 à 11, dans lequel R correspond au groupe méthoxy et Y représente le reste d'histidine.

**20.** Un fragment se composant d'un polymère selon la formule générale IV selon les demandes de brevet 9 à 11, dans lequel R correspond au groupe méthoxy et Y représente le reste d'isoleucine.

**21.** Un fragment se composant d'un polymère selon la formule générale IV selon les demandes de brevet 9 à 11, dans lequel R correspond au groupe méthoxy et Y représente le reste de leucine.

**22.** Un fragment se composant d'un polymère selon la formule générale IV selon les demandes de brevet 9 à 11, dans lequel R correspond au groupe méthoxy et Y représente le reste de lysine.

**23.** Un fragment se composant d'un polymère selon la formule générale IV selon les demandes de brevet 9 à 11, dans lequel R correspond au groupe méthoxy et Y représente le reste de méthionine.

**24.** Un fragment se composant d'un polymère selon la formule générale IV selon les demandes de brevet 9 à 11, dans lequel R correspond au groupe méthoxy et Y représente le reste de phénylalanine.

**25.** Un fragment se composant d'un polymère selon la formule générale IV selon les demandes de brevet 9 à 11, dans lequel R correspond au groupe méthoxy et Y représente le reste de proline.

**26.** Un fragment se composant d'un polymère selon la formule générale IV selon les demandes de brevet 9 à 11, dans lequel R correspond au groupe méthoxy et Y représente le reste de sérine.

**27.** Un fragment se composant d'un polymère selon la formule générale IV selon les demandes de brevet 9 à 11, dans lequel R correspond au groupe méthoxy et Y représente le reste de thréonine.

**28.** Un fragment se composant d'un polymère selon la formule générale IV selon les demandes de brevet 9 à 11, dans lequel R correspond au groupe méthoxy et Y représente le reste de tryptophane.

**29.** Un fragment se composant d'un polymère selon la formule générale IV selon les demandes de brevet 9 à 11, dans lequel R correspond au groupe méthoxy et Y représente le reste de tyrosine.

**30.** Un fragment se composant d'un polymère selon la formule générale IV selon les demandes de brevet 9 à 11, dans lequel R correspond au groupe méthoxy et Y représente le reste de valine.

**31.** Fragments se composant d'un polymère selon la formule générale IV selon les demandes de brevet 12 à 30, dans lesquels les groupes fonctionnels des acides aminés $\alpha$ résiduels Y sont bloqués par des groupes protecteurs.

**32.** Fragments se composant d'un polymère selon la formule générale IV selon les demandes de brevet 12 à 30, dans lesquels les extrémités amines (Nt) des acides aminés résiduels Y sont bloqués par le groupement fluorénylméthyloxycarbonyle.

**33.** Procédé de préparation d'un fragment se composant d'un polymère selon la formule générale IV, caractérisé par l'estérification d'une matrice représentant une résine polymérique selon la formule générale I,

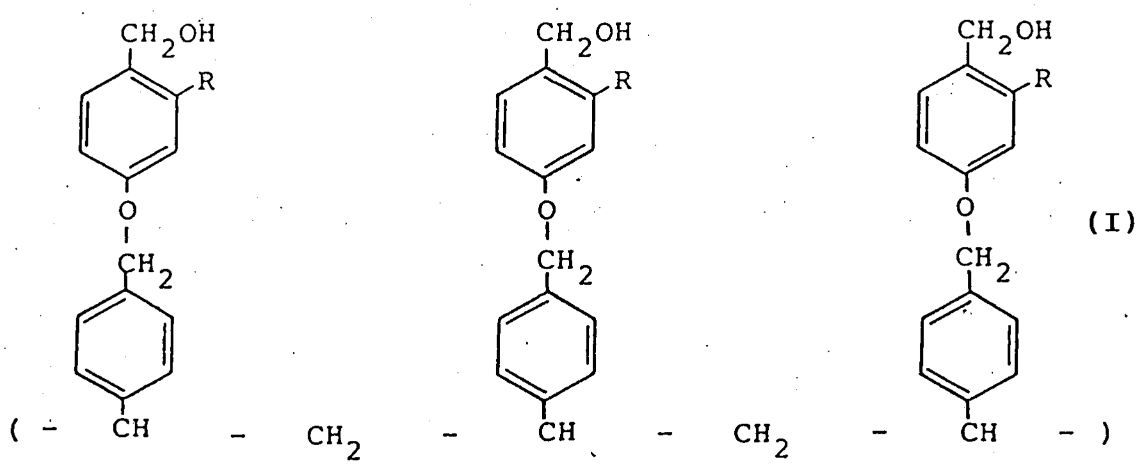

(I)

dans laquelle R représente soit un groupe alkoxy, soit un groupe alkylthio, soit le groupe

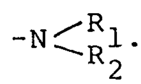

$R_1$ et $R_2$ correspondent à un reste alkyle contenant jusqu'à sept atomes de carbone, de préférence jusqu'à quatre atomes de carbone. Le polymère représenté sur la formule générale I peut être lié transversalement par ajout de 0.5 à 2 % d'un divinylbenzène qui est substitué soit par un halogène soit par un groupe alkyle. Le composé carboxylique nécessaire à l'estérification se compose d'un acide-aminé dont l'extrémité amine (Nt), de même que les autres groupements fonctionnels, s'il y en a, sont protégés par des groupements protecteurs, tandis que le groupe carboxylique reste libre pour l'estérification.

**34.** Procédé selon la demande de brevet 33, qui caractérise l'étape d'estérification selon le procédé d'activation du groupement carboxylique.

**35.** L'utilisation de la combinaison bras-résine décrite sous les demandes de brevet 1 à 3 pour la synthèse de peptides.

**36.** L'emploi des fragments décrits sous les demandes de brevet 9 à 32 pour la synthèse de peptides.